# EUROPEAN PATENT APPLICATION

(11) **EP 4 494 548 A1**
(43) Date of publication of application: **22.01.2025**
(21) Application number: 23461631.6
(22) Date of filing: 21.07.2023
(51) Int. Cl.: A61B 5/00, A61B 5/107

(54) **METHOD FOR DETERMINING THE EFFECT OF A TEST SUBSTANCE ON ENDOTHELIAL FUNCTION**

(71) Applicant: UNIWERSYTET JAGIELLONSKI, 31-007 Kraków (PL)
(72) Inventor: Chlopicki, Stefan, 31-138 Kraków (PL); Bar, Anna, 30-724 Kraków (PL); Marczyk, Brygida, 32-095 Iwanowice (PL); Kwiatkowski, Grzegorz, 30-523 Kraków (PL)
(74) Representative: Witek, Rafal

(57) **Abstract**

A method of determining the effect of a test substance on endothelial function is disclosed, suitable for clinical safety assessment of drugs or drug candidates based on the analysis of their effect on the vascular endothelium *in vivo.*

## Description

The invention relates to a method for determining the effect of a test chemical compound or drug on endothelial function based on a quantitative assessment of the vasorelaxation capacity of blood vessels under *in vivo* conditions. The method is particularly suitable for assessing the vascular safety of drugs or drug candidates based on the analysis of their effects on the vascular endothelium *in vivo.* The proposed method may find application in the pharmaceutical industry, particularly in studies aimed at developing new drugs and determining their pharmacological activities.

The endothelium is a highly specialised organ of the body (Gryglewski, 2005) formed from a single layer of cells lining all blood vessels. Endothelial cells exhibit physiological activities that are important for the function of the cardiovascular system, including the regulation of blood flow and the regulation of thrombotic and inflammatory processes in the blood vessel wall. Endothelial cells secrete a number of biologically active substances such as nitric oxide (NO), prostacyclin and many others (Davignon and Ganz, 2004).

Endothelial dysfunction occurs in the course of many diseases and is characterised by impaired NO-dependent endothelial function, which results in serious vascular complications such as hypertension, atherosclerosis and its cardiovascular complications, including myocardial infarction (Schächinger, Britten and Zeiher, 2000; Halcox *et al.,* 2002). At the same time, the vascular endothelium is influenced by most drugs taken systemically. It is known that endothelial cell function is impaired by some drugs, although this phenomenon is not yet fully investigated. A number of examples from the pharmaceutical industry (e.g. torcetrapib, rofecoxib) demonstrate that adverse drug effects on the endothelium and blood vessel wall resulting in impairment of the vasoprotective role of the vascular endothelium can lead to spectacular failure in drug development and are discovered as late as in clinical trials and lead to post-marketing drug withdrawal.

The existing method of assessing vascular endothelial function in humans by determining flow-mediated vasodilation (FMD) of the brachial artery (Joannides *et al.,* 1995), although recognised as the gold standard of this type of study, is not widely used in clinical practice, and is merely a method used in exploratory studies aimed at assessing the risk of cardiovascular disease, or understanding the mechanisms of disease progression.

At the same time, there is currently no well-documented way to assess the effects of chemical compounds or drug candidates on phenotype of the endothelium in animal models at the preclinical research stage, the results of which detecting adverse effects on endothelial function would be confirmed by clinical observations.

The aim of the invention is to provide a method that would allow the identification and quantification of the level of adverse effects of test substances on vascular endothelium under *in vivo* conditions, based on a quantitative assessment of the vasorelaxation capacity of blood vessels. On the basis of the studies performed, we claim that this method can predict the endotheliotoxic effects of chemical compounds and drugs that may become apparent in clinical trials.

The subject of the invention is a method for determining the effect of a test substance on endothelial function, characterised in that:
a) a dose of the test substance is administered to the mammal,
b) the diameter of a selected artery of the mammal is measured,
c) a vasorelaxant agent is used to induce the response in the selected artery of the mammal,
d) the diameter of the same artery of the mammal is measured again,
e) the effect of the test substance on the endothelium is determined from the magnitude of the endothelium-dependent response, whereby the percentage change in cross-sectional area of the artery after application of the vasorelaxant agent is calculated and this value is considered as a measure of the effect of the test substance on endothelial function.

Preferably, acetylcholine, cevimeline or another M3 receptor agonist is used in step c) as the vasorelaxant agent in the selected mammalian artery at a dose that causes transient dilation of that artery in a healthy mammal.

Preferably, the vasorelaxation induced in step e) by the M3 receptor agonist:
- of a value of 5% and above is considered indicative of no negative effect of the test substance on normal endothelial function;
- of a value of less than 5% is considered indicative of the ability of the test substance to induce mild impairment of endothelial function at the dose administered;
whereas vasoconstriction induced by the M3 receptor agonist:
- of a value of less than 5% is considered indicative of the ability of the test substance to induce moderate impairment of endothelial function at the dose administered;
- of a value of 5% and greater is considered indicative of the ability of the test substance to induce severe impairment of endothelial function at the dose administered.

Preferably, the selected artery is the aorta, preferably the abdominal aorta for assessment of the endothelium-dependent response to acetylcholine administration.

Preferably, step d) is performed 30 minutes after step c) for assessment of the response to M3 receptor agonist administration.

Preferably, acetylcholine is administered to the animal intraperitoneally, particularly preferably at a dose in the range 5-16 mg/kg.

Preferably, cevimeline is administered to the animal or human orally or intravenously at a dose in the range 2-18 mg/kg.

In another preferred variant of the embodiment, increased blood flow induced by first closing the blood flow through the blood vessel for 2-5 minutes is used in step c) to induce vasorelaxation of the selected artery of the mammal.

Preferably, the vasorelaxation induced in step e) by increased blood flow:
- of a value of 25% and greater is considered to be indicative of no negative effect of the test substance on normal endothelial function;
- of a value less than 25% but greater than 20% is considered indicative of the ability of the test substance to induce mild impairment of endothelial function at the dose administered;
- of a value of less than 20% but greater than 10% is considered indicative of the ability of the test substance to induce moderate impairment of endothelial function at the dose administered;
- of a value of less than 10% is considered indicative of the ability of the test substance to induce severe impairment of endothelial function at the dose administered.

Preferably, the selected artery is the femoral artery for assessing the endothelium-dependent response to increased blood flow.

Preferably, step d) is performed 5 minutes after step c) for assessment of the response to increased blood flow.

Preferably, the mammal is a rodent, particularly preferably a mouse.

In the method of the invention, any mammal can be tested. In particular, it can be: the house mouse (*Mus musculus*), the brown rat (*Rattus norvegicus*), the guinea pig (*Cavia porcellus*), the golden hamster (*Mesocricetus auratus*), the Mongolian gerbil (*Meriones unguiculatus*), the European rabbit (*Oryctolagus cuniculus*), domestic pig (*Sus domestica*), domestic dog (*Canis lupus familiaris*), the rhesus macaque (*Macaca mulatta*), human (*Homo sapiens*) or any other mammal.

Preferably, the selected artery is the aorta, particularly preferably the abdominal aorta.

In the method of the invention, any artery or segment thereof may be examined, except the basilar artery (Latin: *arteria basilaris*). In particular, it can be the ascending aorta, the aortic arch, the thoracic (descending) aorta, the abdominal aorta, the axillary artery, the brachial artery, the ulnar artery, the radial artery, the profundal artery, the femoral artery, the iliac artery, the popliteal artery, the carotid artery or any arterial vessel except the basilar artery (Latin: *arteria basilaris*).

Preferably, the diameter of the artery is measured by magnetic resonance imaging (MRI). It is also possible to use any other known method that allows imaging and measurement of the artery diameter. Beyond MRI, this may include optical coherence tomography (OCT) imaging, ultrasound imaging (ultrasonography) or any other technique that allows arterial imaging.

Preferably, vasodilation of a blood vessel is induced by brief occlusion of that vessel and vasorelaxation is assessed after release of the occlusion when evaluating the response to increased blood flow.

Preferably, the test substance is a drug or drug candidate, and particularly preferably, the dose administered in step a) is the dose considered to be the optimal therapeutic dose in humans, considering conversions of the dose into animal dose, according to generally accepted schemes for such conversions.

The proposed method of studying the vascular endothelium *in vivo* using magnetic resonance imaging allows a quantitative preclinical assessment of drug effects on the vascular endothelium and enables relative vascular safety profiling of drug candidates. In the embodiment, the results of an assessment of the endothelial effects of several drugs performed by the proposed method of assessing their endothelial toxicity are presented, which can be the basis for predicting adverse drug effects on the vascular endothelium and consequently predicting cardiovascular complications induced by the evaluated drug in the clinical setting.

In order to better explain the invention, it has been presented in the embodiments described below, which, however, should not be equated with the essence of the invention as defined above.

At the same time, the embodiments of the invention are illustrated by the accompanying figures.

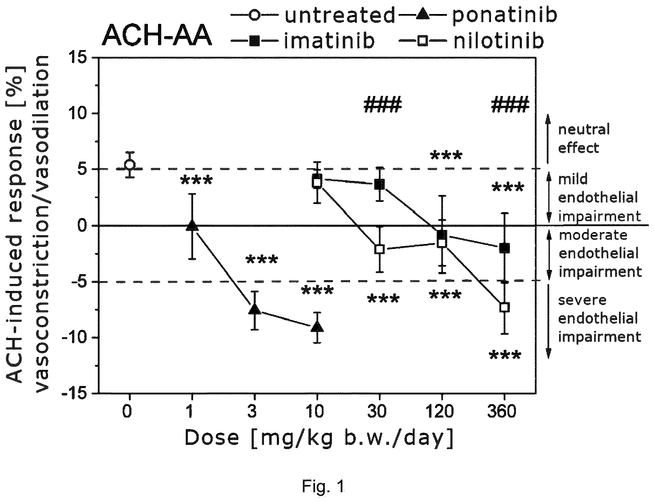

Fig. 1 shows the dose-dependent effects of imatinib, nilotinib and ponatinib (tyrosine kinase inhibitors - TKIs) on the endothelium-dependent vascular response to acetylcholine administration in young mice (10-12 weeks of age) of C57BL/6 strain. Endothelium-dependent function assessed by the change in abdominal aortic volume (ACH-AA) assessed 30 minutes after acetylcholine (Ach) administration. Endothelium-dependent responses in C57BL/6 mice treated with the above-mentioned TKI drugs for 4 weeks (measured at 14-16 weeks of age) were compared with corresponding responses in untreated C57BL/6 mice (n=5-6, Control). Statistics: two-way ANOVA, Tukey's post hoc test (normality assessed using the Shapiro-Wilk test), *** p <0.001 for mice treated with imatinib, nilotinib and ponatinib compared with untreated mice and ### p <0.001 for imatinib compared with nilotinib.

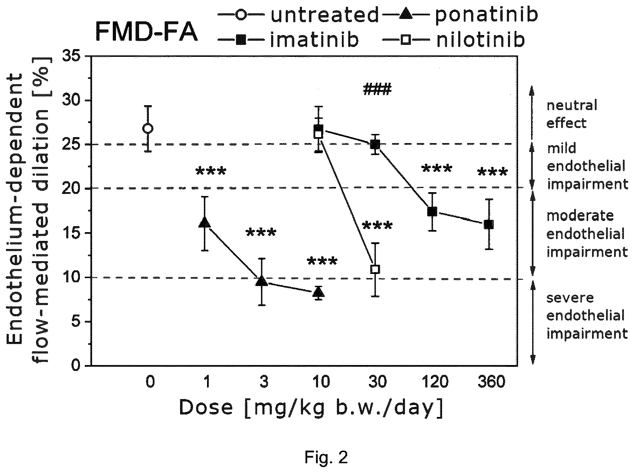

Fig. 2 shows the dose-dependent effects of imatinib, nilotinib and ponatinib on the endothelium-dependent vascular response induced by increased blood flow (FMD) in young mice (10-12 weeks old) of strain C57BL/6. Endothelium-dependent function was assessed by the change in femoral artery volume (FMD-FA) after a 5-minute vessel occlusion. Endothelium-dependent responses in C57BL/6 mice treated with the above-mentioned TKI drugs for 4 weeks (measured at 14-16 weeks of age) were compared with corresponding responses in untreated C57BL/6 mice (n=5-6, Control). Statistics: two-way ANOVA, Tukey's post hoc test (normality assessed using the Shapiro-Wilk test), *** p <0.001 for mice treated with imatinib, nilotinib and ponatinib compared with untreated mice and ### p <0.001 for imatinib compared with nilotinib.

### Example 1. Method to assess the endothelium-dependent response of the abdominal aorta to acetylcholine administration.

Description of the method used: *In vivo* studies were performed in mice using magnetic resonance imaging (9.4 T MRI scanner, BioSpec 94/20 USR, Bruker, Germany). The *in vivo* study was conducted in a mouse (lying supine) under inhalation anaesthesia with isoflurane at a concentration of 1.5-1.75% in a 1:2 mixture of oxygen and air, with simultaneous monitoring of the animal's condition using a respiratory sensor, a temperature sensor (maintaining temperature at 37°C with circulating warm water) and an ECG system (SA Inc., Stony Brook, USA).

Description of the technique used: The assessment of vascular endothelial function was based on evaluation of the endothelium-dependent response of the abdominal aorta (AA) 30 minutes after administration of acetylcholine (Ach, Sigma-Aldrich, Poznań, Poland: 50 µL, 16.6 mg/kg, *i.p.*). In a healthy individual, Ach induces aortic vasorelaxation, whereas in the case of impairment of endothelial function, induced in particular by a previously administered dose of the drug under test, the vasorelaxation is reduced or the response to Ach may even change to vasoconstriction.

Responses in AA were studied by comparing two 3D images at maximal vessel relaxation, before and 30 minutes after Ach administration. These images were acquired using IntraGate FLASH 3D cine sequences and reconstructed using IntraGate 1.2.b.2 macro (Bruker). Vascular volume analysis was performed using Imaged 1.46r software (NIH Bethesda, Maryland, USA).

The level of endothelium-dependent response was determined by calculating the percentage change in the volume of the artery after acetylcholine administration in mice that had previously been administered various drugs, particularly those leading to impairment of vascular endothelial function.

As a result of these studies, a scale was established to determine the effect of the test substance on endothelial function, according to which:
- vasorelaxation of 5% in value and greater is considered indicative of no negative effect of the test substance on normal endothelial function;
- vasorelaxation of less than 5% in value is considered indicative of the ability of the test substance to induce mild impairment of endothelial function at the dose administered;
- vasoconstriction of less than 5% in value is considered indicative of the ability of the test substance to induce moderate impairment of endothelial function at the dose administered;
- vasoconstriction of 5% in value and greater is considered indicative of the ability of the test substance to induce severe impairment of endothelial function at the dose administered.

### Example 2. Endothelial profiling of three tyrosine kinase inhibitor drugs using assessment of the endothelium-dependent response to acetylcholine administration.

A study was performed to assess the endothelium-dependent response of the abdominal aorta to acetylcholine (Ach) administration, using the method described in Example 1, in mice treated with three known tyrosine kinase inhibitor (TKI) drugs.

### Description of the TKIs used and their doses:

The drugs used were imatinib (1^{st} generation TKI drug), nilotinib (2^{nd} generation TKI drug) and ponatinib (3^{rd} generation TKI drug). Experimental mice were administered the tested drugs in the following dose range: ponatinib - 1-10 mg/kg, nilotinib, imatinib - 10-360 mg/kg.

The graph presented (Fig.1) shows that ponatinib administered at relevant therapeutic doses in an *in vivo* animal model induced severe endothelial dysfunction, nilotinib and imatinib induced moderate endothelial dysfunction. Endothelial dysfunction measured *in vivo* using the method described in Example 1 was therefore more severe for the 3^{rd} generation tyrosine kinase inhibitor (ponatinib) compared to 2^{nd} generation inhibitors (nilotinib), while the 1^{st} generation inhibitor (imatinib) had the least effect on endothelial function and these results were presented on a quantitative scale.

### Example 3. Method to assess the endothelium-dependent response of the femoral artery induced by increased blood flow.

The method described in Example 1 was used in mice.

Description of the technique used: The assessment of vascular endothelial function was based on the evaluation of the endothelium-dependent response of the femoral artery (FA) to increased blood flow (i.e. flow-mediated dilation, FMD) induced by a 5-minute vessel occlusion, as described by Sternak *et al.* 2018. In a healthy individual, temporary vasodilation of the femoral artery occurs in response to the increased blood flow, whereas there is less dilation when the vascular endothelium is impaired, induced in particular by a previously administered dose of the test drug.

Responses in FA were studied by comparing two 3D images at vessel relaxation, before and after the 5-minute vessel occlusion. These images were acquired using IntraGate FLASH 3D cine sequences and reconstructed using IntraGate 1.2.b.2 macro (Bruker). Vascular volume analysis was performed using Imaged 1.46r software (NIH Bethesda, Maryland, USA).

The level of endothelium-dependent response was determined by calculating the percentage change in the volume of that artery in response to increased blood flow, in mice that had previously been administered various drugs, particularly those leading to impairment of vascular endothelial function.

As a result of these studies, a scale was established to determine the effect of the test substance on endothelial function, according to which:
- vasorelaxation of 25% in value and greater is considered indicative of no negative effect of the test substance on normal endothelial function;
- vasorelaxation of less than 25% in value but more than 20% is considered indicative of the ability of the test substance to induce mild impairment of endothelial function at the dose administered;
- vasorelaxation of less than 20% in value but more than 10% is considered indicative of the ability of the test substance to induce moderate impairment of endothelial function at the dose administered;
- vasorelaxation of less than 10% in value is considered indicative of the ability of the test substance to induce severe impairment of endothelial function at the dose administered.

### Example 4. Endothelial profiling of three tyrosine kinase inhibitor drugs using assessment of the endothelium-dependent response to increased blood flow (FMD).

A study was performed to assess the endothelium-dependent response of the femoral artery to increased blood flow, using the method described in Example 3, in mice treated with three known tyrosine kinase inhibitor (TKI) drugs in the following dose range (ponatinib - 1-10 mg/kg, nilotinib, imatinib - 10-360 mg/kg).
owing dose range: ponatinib - 1-10 mg/kg, nilotinib, imatinib - 10-360 mg/kg.

The graph presented (Fig. 2) shows that, at the relevant therapeutic doses in the *in vivo* animal model, ponatinib induced severe endothelial dysfunction, while nilotinib and imatinib induced moderate endothelial dysfunction. Endothelial dysfunction measured *in vivo* using the method described in Example 3 was therefore more severe with the 3^{rd} generation tyrosine kinase inhibitor (ponatinib) compared to 2^{nd} generation inhibitors (nilotinib), while the 1^{st} generation inhibitor (imatinib) had the least effect on endothelial function and these results were presented on a quantitative scale.

### Conclusions:

The two variants of the method to evaluate endothelial effects of drugs based on their effect on the endothelium-dependent vasorelaxation response of the artery allow the prediction of cardiovascular complications in patients resulting from adverse endothelial effects of the drugs used.

Indeed, the results obtained are fully consistent with clinical data obtained when assessing the vascular safety of the same TKI inhibitors tested in humans (Moslehi and Deininger, 2015; Novo *et al.,* 2020), and the results of these studies, which assessed the risk of cardiovascular complications and the incidence of cardiovascular complications for imatinib, nilotinib and ponatinib, are presented in the table below.

Thus, the described invention allows for evaluating the adverse effects of the tested chemical compounds and drugs on the vascular endothelium *in vivo,* in preclinical studies, in experimental animals based on a quantitative assessment of the vasorelaxation capacity of blood vessels, and the results obtained have predictive value for the adverse cardiovascular effects of therapy in humans that result from endothelial dysfunction induced by the drugs used.

| Drug studied | 6-year incidence of cardiovascular events* | Hypertension | Abnormal ankle-brachial index (ABI)* | Overall cardiovascular risk | Preclinical endothelial profiling |
|---|---|---|---|---|---|
| Imatinib (1st generation) | 2.5% (400 mg once per day) | N/A | 6.3% (first-line imatinib) | low | endothelial dysfunction detected in mice at a HIGH DAILY DOSE |
| Nilotinib (2nd generation) | 15.9% (400 mg twice per day) | 6.6%** | 26% (first-line nilotinib) | medium | endothelial dysfunction detected in mice at a MEDIUM DAILY DOSE |
| Ponatinib (3rd generation) | cumulative serious adverse effects: 10% cardiovascular, 7% cerebrovascular, 7% peripheral, 14% arterial, 3% venous (median follow-up 28 months) | 26% (median follow-up 28 months)* | N/A (first-line trial closed due to significant vascular toxicity) | high | endothelial dysfunction detected in mice at a LOW DAILY DOSE |

| | | | | | |
|---|---|---|---|---|---|
| * Moslehi & Deininger (2015) J Clin Oncol 33:4210-4218; ** Novo et al. (2020) Oncology 98(7):445-451 | | | | | |

### Literature:

Davignon, J. and Ganz, P. (2004) 'Role of endothelial dysfunction in atherosclerosis.', Circulation, 109(23 Suppl 1), pp. III27-32. doi: 10.1161/01.CIR.0000131515.03336.f8.
Gryglewski, R. J. (2005). "Pharmacology of vascular endothelium. FEBS J 272(12): 2956-2967.
Halcox, J. P. J. et al. (2002) 'Prognostic value of coronary vascular endothelial dysfunction.', Circulation, 106(6), pp. 653-8. Available at: http://www.ncbi.nlm.nih.gov/pubmed/12163423 (Accessed: 7 February 2017).
Joannides, R. et al. (1995) `Nitric oxide is responsible for flow-dependent dilatation of human peripheral conduit arteries in vivo.', Circulation, 91(5), pp. 1314-9.
Moslehi, J. J. and Deininger, M. (2015) 'Tyrosine kinase inhibitor-associated cardiovascular toxicity in chronic myeloid leukemia', Journal of Clinical Oncology, 33(35), pp. 4210-4218. doi: 10.1200/JCO.2015.62.4718.
Novo, G. et al. (2020) `Cardiovascular Toxicity in Cancer Patients Treated with Tyrosine Kinase Inhibitors: A Real-World Single-Center Experience', Oncology (Switzerland). S. Karger AG, 98(7), pp. 445-451. doi: 10.1159/000505486.
Schächinger, V., Britten, M. B. and Zeiher, A. M. (2000) 'Prognostic impact of coronary vasodilator dysfunction on adverse long-term outcome of coronary heart disease.', Circulation, 101(16), pp. 1899-906. Available at: http://www.ncbi.nlm.nih.gov/pubmed/10779454 (Accessed: 7 February 2017).
Sternak M, Bar A, Adamski MG, Mohaissen T, Marczyk B, Kieronska A, Stojak M, Kus K, Tarjus A, Jaisser F, Chlopicki S. The Deletion of Endothelial Sodium Channel α (αENaC) Impairs Endothelium-Dependent Vasodilation and Endothelial Barrier Integrity in Endotoxemia in Vivo. Front Pharmacol. 2018 Apr 10;9:178. doi: 10.3389/fphar.2018.00178. eCollection 2018.

## Claims

1. A method for determining the effect of a test substance on endothelial function, **characterised in that**:
a) a dose of the test substance is administered to the mammal,
b) the diameter of a selected artery of the mammal is measured,
c) a vasorelaxant agent is used to induce the response in the selected artery of the mammal,
d) the diameter of the same artery of the mammal is measured again,
e) the effect of the test substance on the endothelium is determined from the magnitude of the endothelium-dependent response, whereby the percentage change in cross-sectional area of the artery after application of the vasorelaxant agent is calculated and this value is considered as a measure of the effect of the test substance on endothelial function.

2. The method of claim 1, **characterised in that** acetylcholine, cevimeline or another M3 receptor agonist is used in step c) as the vasorelaxant agent in the selected mammalian artery at a dose that causes transient dilation of that artery in a healthy mammal.

3. The method of claim 2, **characterised in that** the vasorelaxation induced in step e) by the M3 receptor agonist:
- of a value of 5% and above is considered indicative of no negative effect of the test substance on normal endothelial function;
- of a value of less than 5% is considered indicative of the ability of the test substance to induce mild impairment of endothelial function at the dose administered;
whereas vasoconstriction induced by the M3 receptor agonist:
- of a value of less than 5% is considered indicative of the ability of the test substance to induce moderate impairment of endothelial function at the dose administered;
- of a value of 5% and greater is considered indicative of the ability of the test substance to induce severe impairment of endothelial function at the dose administered.

4. The method of claim 2, **characterised in that** the selected artery is the aorta, preferably the abdominal aorta for assessment of the endothelium-dependent response to acetylcholine administration.

5. The method of claim 2, **characterised in that** step d) is performed 30 minutes after step c) for assessment of the endothelium-dependent response to acetylcholine administration.

6. The method of claim 2, **characterised in that** acetylcholine is administered intraperitoneally, preferably at a dose in the range 5-16 mg/kg.

7. The method of claim 1, **characterised in that** increased blood flow is used in step c) as a factor to induce vasorelaxation of the selected artery of the mammal.

8. The method of claim 7, **characterised in that** the vasorelaxation induced in step e) by increased blood flow:
- of a value of 25% and greater is considered to be indicative of no negative effect of the test substance on normal endothelial function;
- of a value less than 25% but greater than 20% is considered indicative of the ability of the test substance to induce mild impairment of endothelial function at the dose administered;
- of a value of less than 20% but greater than 10% is considered indicative of the ability of the test substance to induce moderate impairment of endothelial function at the dose administered;
- of a value of less than 10% is considered indicative of the ability of the test substance to induce severe impairment of endothelial function at the dose administered.

9. The method of claim 7, **characterised in that** the selected artery is the femoral artery for assessing the endothelium-dependent response to increased blood flow.

10. The method of claim 7, **characterised in that** step d) is performed 5 minutes after step c) for assessment of the response to increased blood flow.

11. The method of claim 1, **characterised in that** the mammal is a rodent, preferably a mouse.

12. The method of claim 1, **characterised in that** the diameter of the artery is measured by magnetic resonance imaging.

13. The method of claim 1, **characterised in that** the test substance is a drug or a drug candidate.

14. The method of claim 13, **characterised in that** a dose administered in step a) is a dose which falls within the therapeutic range of doses of the test drug.
